# EUROPEAN PATENT APPLICATION

(11) **EP 4 759 339 A1**
(43) Date of publication of application: **17.06.2026**
(21) Application number: 25222703.8
(22) Date of filing: 11.12.2025
(51) Int. Cl.: A61M 1/16

(54) **MANAGEMENT METHODS, SYSTEMS, AND MEDIA FOR PRESSURE STABILIZATION OF EXTRACORPOREAL MEMBRANE OXYGENATION SYSTEMS**

(30) Priority: 13.12.2024 CN 202411841358
(71) Applicant: Lifemotion Medical Technology Co., Ltd., Shenzhen, Guangdong 518102 (CN)
(72) Inventor: ZHANG, Wenyi, Shenzen Guangdong 518102 (CN); LIU, Bisheng, Shenzen Guangdong 518102 (CN)
(74) Representative: Pfenning, Meinig & Partner mbB

(57) **Abstract**

A management method, system, and medium for pressure stabilization of an extracorporeal membrane oxygenation (ECMO) system is provided. The method comprises turning on a switch for the pressure stabilization mode of the extracorporeal membrane oxygenation system, determining whether conditions for entering the pressure stabilization mode are met, and if so, entering the pressure stabilization mode. The method further comprises obtaining a speed change of a power source of the extracorporeal membrane oxygenation system, a flow change of a flow detector of the extracorporeal membrane oxygenation system, and a pressure change of a pressure detector of the extracorporeal membrane oxygenation system, and adjusting the speed of the power source of the extracorporeal membrane oxygenation system to a target speed based on the speed change, flow change, and pressure change, the target speed being used to maintain the flow and pressure within a safe range.

## Description

### CROSS REFERENCE TO RELATED APPLICATION

The present application claims priority to Chinese Patent Application No. 202411841358.7, filed December 13, 2024, the disclosure of which is incorporated herein in its entirety.

### TECHNICAL FIELD

The embodiments of the present application belong to the field of medical equipment technology, and in particular, relate to a management method, system, and medium for a pressure stabilization mode of an extracorporeal membrane oxygenation system.

### BACKGROUND ART

To make extracorporeal membrane oxygenation (ECMO) systems more intelligent, numerous medical device companies are developing auxiliary functions such as automatic regulation. However, the current automatic blood pressure regulation mechanism is not yet fully developed, and there is a risk of complications such as hemolysis caused by abnormal blood flow caused by speed regulation. This is because the automatic pressure stabilization mode is based solely on the comparison of the value of a pressure detector, such as a pressure sensor, with a predefined safety value. However, the pressure within the ECMO system circuit is often affected by many factors. Relying solely on pressure as a criterion is insufficient and carries a relatively high risk. Furthermore, the entry, management, and exit of the automatic regulation mechanism are generally based on the relationship between the pressure reading and its set safety value. If it is contrary the safety value, the system enters automatic regulation mode, gradually adjusting the motor speed until the pressure is in compliance with the set safety value. However, if the pressure detector malfunctions, the recorded pressure value remains unchanged while the motor speed continues to change. This can cause the actual pressure value to be too high or too low, potentially causing irreversible harm or even death to the patient.

### SUMMARY OF THE INVENTION

The present application provides a management method, system, and computer-readable storage medium for the pressure stabilization mode of an extracorporeal membrane oxygenation system, which can solve the problem mentioned above of adjusting the motor speed of the ECMO system only according to the pressure value recorded by a pressure detector, resulting in excessively high or low pressure values and thus posing safety risks.

To achieve the above objectives, the present application adopts the following technical solutions:
In a first aspect, a method for managing a pressure stabilization mode of an extracorporeal membrane oxygenation system is provided, the method comprising:
turn on the switch for the pressure stabilization mode of the extracorporeal membrane oxygenation system;
determine whether the conditions for entering the pressure stabilization mode are met, and if so, enter the pressure stabilization mode;
obtaining a rotation speed change of a power source of the extracorporeal membrane oxygenation system, a flow rate change of a flow detector of the extracorporeal membrane oxygenation system, and a pressure change of a pressure detector of the extracorporeal membrane oxygenation system;
the speed of the power source of the extracorporeal membrane oxygenation system is adjusted to a target speed according to the speed change, the flow change, and the pressure change. The target speed is used to keep the flow and pressure within a safe range.

In one possible implementation, the conditions for entering the pressure stabilization mode include: a switch for the pressure stabilization mode is turned on, and at least one of the following is satisfied: a pressure value of a pre-pump pressure detector of the extracorporeal membrane oxygenation system is less than a first safety value, a pressure value of a pre-membrane pressure detector of the extracorporeal membrane oxygenation system is greater than a second safety value, and a pressure value of a post-membrane pressure detector of the extracorporeal membrane oxygenation system is greater than a third safety value.

In one possible implementation, adjusting the speed of a power source of the extracorporeal membrane oxygenation system to a target speed based on the speed change, the flow change, and the pressure change includes:
determining that the speed change is greater than or equal to a speed threshold, and that the speed change is positively correlated with the flow change and the pressure change, respectively;
determining that the flow rate change is greater than or equal to a flow rate threshold, and the pressure change is greater than or equal to a pressure threshold;
performing proportional-integral-differential adjustments according to a pressure value and a safety value recorded by at least one of a pre-pump pressure detector, a pre-membrane pressure detector, and a post-membrane pressure detector of the extracorporeal membrane oxygenation system to generate a first rotational speed;
if the first speed is greater than or equal to the minimum load speed, setting the first speed as the target speed, the minimum load speed being determined according to the load capacity of the power source in the extracorporeal membrane oxygenation system and the expected load;
the target speed is sent to the control component of the power source.

Optionally, the speed threshold is selected from a hydraulic characteristic curve of the extracorporeal membrane oxygenation system and is a minimum load speed change that causes a recognizable change in pressure under any state.

Optionally, the pressure change is the difference between a first pressure value recorded by at least one of the pre-pump pressure detector, pre-membrane pressure detector, and post-membrane pressure detector of the extracorporeal membrane oxygenation system after the speed of the power source of the extracorporeal membrane oxygenation system is adjusted using the target speed, and a second pressure value recorded by at least one of the pre-pump pressure detector, pre-membrane pressure detector, and post- membrane pressure detector of the extracorporeal membrane oxygenation system before the speed of the power source of the extracorporeal membrane oxygenation system is adjusted using the target speed.

Optionally, the flow rate change is a difference between a first flow rate value recorded by a flow detector of the extracorporeal membrane oxygenation system after the speed of the power source of the extracorporeal membrane oxygenation system is adjusted using the target speed, and a second flow rate value recorded by the flow detector of the extracorporeal membrane oxygenation system before the speed of the power source of the extracorporeal membrane oxygenation system is adjusted using the target speed.

In one possible implementation, adjusting the speed of a power source of the extracorporeal membrane oxygenation system to a target speed based on the speed change, the flow change, and the pressure change includes:
determining that the speed change is less than a speed threshold;
generating a second rotational speed;
if the second speed is greater than or equal to the minimum load speed, the second speed is set as the target speed, and the minimum load speed is determined according to the load capacity of the power source of the extracorporeal membrane oxygenation system and the expected load;
a target speed is sent to a control component of a power source of the extracorporeal membrane oxygenation system.

Furthermore, the method further comprises:
if one or more of the following conditions are met, the system exits the pressure stabilization mode or does not enter the pressure stabilization mode:
the flow detector of the extracorporeal membrane oxygenation system is faulty or not connected;
the pressure detector of the extracorporeal membrane oxygenation system is faulty or not connected;
the flow rate change is less than the flow rate threshold for M consecutive times, where M is a positive integer and M>1;
the pressure change is less than the pressure threshold for N consecutive times, where N is a positive integer and N>1;
the power source of the extracorporeal membrane oxygenation system is not operating;
the pressure value recorded by the pre-pump pressure detector of the extracorporeal membrane oxygenation system is greater than or equal to the first safety value;
the pressure value recorded by the pre- membrane pressure detector of the extracorporeal membrane oxygenation system is less than or equal to the second safety value;
the pressure value recorded by the post-membrane pressure detector of the extracorporeal membrane oxygenation system is less than or equal to the third safety value;
an instruction to intervene in the rotation speed of the power source of the extracorporeal membrane oxygenation system is received from the outside.

In a second aspect, an extracorporeal membrane oxygenation system is provided, comprising a pump, a membrane oxygenator, a flow detector, at least one pressure detector, a processor, and a memory, wherein the memory stores a program or instruction. When the program or instruction is executed by the processor, the extracorporeal membrane oxygenation system executes the method for managing a pressure stabilization mode of an extracorporeal membrane oxygenation system as described in any implementation of the first aspect.

In a third aspect, a computer-readable storage medium is provided, which stores a program or instruction. When a computer reads and executes the program or instruction, the computer executes the method for managing the pressure stabilization mode of the extracorporeal membrane oxygenation system comprising a pump, a membrane oxygenator, a flow detector, and at least one pressure detector as described in any implementation of the first aspect.

Based on the management method, system and computer-readable storage medium of the pressure stabilization mode of the extracorporeal membrane oxygenation system provided in the embodiments of the present application, the target speed can be generated by comprehensively considering the speed change of the power source of the ECMO system and the flow change and pressure change recorded by various detectors. The target speed is used to adjust the motor speed of the ECMO system so as to keep the flow and pressure of the ECMO system within a safe range. This can solve the problem of adjusting the motor speed of the ECMO system only according to the pressure value recorded by the pressure detector, resulting in a pressure value that is too high or too low and thus posing a safety risk, thereby improving the safety of the ECMO system.

In addition, operations such as turning on the ECMO pressure stabilization mode, entering the pressure stabilization mode, and exiting the pressure stabilization mode are also judged according to the hardware working status of the ECMO system and the working status and measurement values of various detectors. The corresponding operations will be performed only when the conditions are met, thereby further improving the safety of the ECMO system.

### BRIEF DESCRIPTION OF THE DRAWINGS

The drawings described herein are used to provide a further understanding of the present application and constitute a part of the present application. The illustrative embodiments of the present application and their descriptions are used to explain the present application and do not constitute an improper limitation of the present application. Some specific embodiments of the present application will be described in detail in an illustrative and non-restrictive manner with reference to the drawings. The same reference numerals in the drawings indicate the same or similar components or parts. It should be understood by those skilled in the art that these drawings are not necessarily drawn to scale. In the drawings:
Figure 1 is a flow chart of a method for managing a pressure stabilization mode of an extracorporeal membrane oxygenation system provided in an embodiment of the present application;
Figure 2 is a schematic diagram of a flow chart of a switch for turning on the P1, P2, or P3 pressure stabilization mode according to an embodiment of the present application;
Figure 3A is a schematic diagram of a process for entering the P1 pressure stabilization mode according to an embodiment of the present application;
Figure 3B is a schematic diagram of a process for entering the P2 (or similarly for entering the P3) pressure stabilization mode provided in an embodiment of the present application.
Figure 4 is a schematic diagram of a process for performing pressure regulation in a P1 pressure stabilization mode according to an embodiment of the present application;
Figure 5A is a schematic diagram of a process for exiting the P1 pressure stabilization mode according to an embodiment of the present application;
Figure 5B is a schematic diagram of a process of exiting the P2 (or similarly for exiting the P3) pressure stabilization mode according to an embodiment of the present application;
Figure 6 is a schematic structural diagram of a control component of an extracorporeal membrane oxygenation system provided in an embodiment of the present application.

### DETAILED DESCRIPTION

In order to enable those skilled in the art to better understand the present invention, the technical solutions in the embodiments of the present invention will be clearly and completely described below in conjunction with the drawings in the embodiments of the present invention. Obviously, the described embodiments are only part of the embodiments of the present invention, not all of the embodiments. Based on the embodiments in the present invention, all other embodiments obtained by ordinary technicians in this field without making creative efforts should fall within the scope of protection of this application.

The technical terms and prior art involved in the technical solutions provided in the embodiments of the present application are introduced.

Pressure and flow values: The pressure and flow values displayed on the display panel are filtered, stable values that are more stable (the stable values can be obtained through one or more processes of the detector's own filtering, driver control filtering, and hub filtering). The detector can be a sensor or other device that can be used to measure one or more physical quantities such as pressure and flow, such as a pressure sensor, flow sensor, etc.

Adjustment frequency: The frequency adjustment can be set in combination with the ECMO system's pipeline length, diameter, load capacity of the power source, and reaction speed. In summary, the time required for the speed to stabilize the flow pressure is obtained through comprehensive compilation of data obtained through repeated experimental tests.

Effective/ineffective regulation: The value for judging whether the regulation is effective is determined by referring to the hydraulic characteristic curve of the ECMO system.

Minimum flow rate: The ECMO user (e.g., a doctor) sets the minimum flow rate value in the ECMO system to maintain the patient's body functions based on the patient's condition.

Speed threshold: This is a value selected based on the hydraulic characteristic curve of the ECMO system. It is the minimum load speed change value that can be recognized by a detector as a pressure or flow change under any state.

Pressure threshold: When the speed change is greater than the speed threshold, the minimum pressure change that can be recognized by the pressure detector can be obtained based on the hydraulic characteristic curve of the ECMO system.

Flow threshold: When the speed change is greater than or equal to the speed threshold, the minimum flow change that can be recognized by the flow detector is obtained based on the hydraulic characteristic curve of the ECMO system.

Minimum load speed: This is defined based on the motor load capacity of the ECMO system's power source and the expected load. This minimum load speed allows for more accurate identification of pressure and flow changes. The power source can be the ECMO system's blood pump or other device that provides power to adjust ECMO pressure and flow, and this embodiment of the present application is not limited thereto.

To determine whether the speed has been adjusted: You can determine whether the speed signal of the setting knob, setting button or setting touch screen changes.

The hydraulic characteristic curve of the ECMO system: It can be obtained by the following steps:
Use animal blood or blood substitutes such as a mixture of ethanol and water or glycerol and water, and use consumables and equipment to build a circulatory system;
According to the speed setting in the record table, adjust different pump speeds, record the corresponding flow rate and pump inlet pressure, pump outlet pressure, and calculate the (pump outlet pressure minus pump inlet pressure) value;
Make a curve diagram of the speed-flow-pressure relationship based on the contents in the table, which is the hydraulic characteristic curve.

Pressure detector: In some embodiments, there are up to three pressure detectors in the ECMO circulation pipeline, namely P1, P2, and P3 detectors. P1 is the pre-pump pressure detector, P2 is the pre-membrane pressure detector, and P3 is the post-membrane pressure detector.

The control method for ECMO pressure stabilization mode includes: after entering pressure stabilization mode, automatically adjusting the speed of the power source (e.g., motor, blood pump) to adjust the pressure value to a safe range. This is intended to ensure patient safety and prevent excessive intratubing pressure from harming the patient. For example, when using the pre-pump pressure value P1 as the criterion for exiting P1 pressure stabilization mode, exit can be achieved if the pre-pump pressure value P1 is greater than or equal to a first safety value. Similarly, when using the pre-membrane pressure value P2 or the post-membrane pressure value P3 as the criterion for exiting pressure stabilization mode, exit can be achieved if the P2 pressure value is less than or equal to a second safety value, or if the P3 pressure value is less than or equal to a third safety value.

The technical solutions provided in the embodiments of the present application are described in detail below with reference to the accompanying drawings.

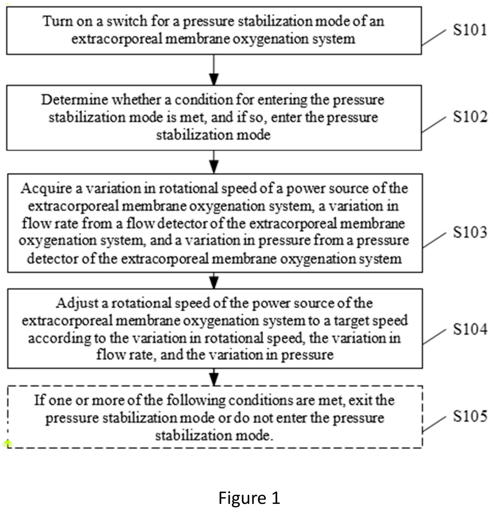

For example, Figure 1 is a flow chart of a method for managing a pressure stabilization mode of an extracorporeal membrane oxygenation system provided in an embodiment of the present application. As shown in Figure 1, the method includes S101-S104:
S101: Turning on the switch for the pressure stabilization mode of the extracorporeal membrane oxygenation system;
S102, Determining whether the conditions for entering the pressure stabilization mode are met, and if so, entering the pressure stabilization mode;
S103, Obtaining a speed change of a power source of the extracorporeal membrane oxygenation system, a flow change of a flow detector of the extracorporeal membrane oxygenation system, and a pressure change of a pressure detector of the extracorporeal membrane oxygenation system;
S104, Adjusting the speed of the power source of the extracorporeal membrane oxygenation system to a target speed based on the speed change, the flow change, and the pressure change. The target speed is used to keep the flow and pressure within a safe range.

For example, in response to the defects of the existing technology and in combination with the situation of the ECMO system, signals such as flow rate, detector status, motor operating status (such as speed) are used as reference conditions for entering, managing, and exiting the pressure stabilization mode.

Assume that there are three pressure detectors in the ECMO device, namely the pre-pump pressure detector P1, the pre-membrane pressure detector P2, and the post-membrane pressure detector P3. To simplify the description, the pressure values recorded by the above pressure detectors are also represented by the same variables, that is, P1 is the pressure value recorded by the pre-pump pressure detector P1 (also known as the pre-pump pressure value P1 or pressure value P1), P2 is the pressure value recorded by the pre-membrane pressure detector P2 (also known as the pre-membrane pressure value P2 or pressure value P2), and P3 is the pressure value P3 recorded by the post-membrane pressure detector (also known as the post-membrane pressure value P3 or pressure value P3). When the negative pressure recorded by the pre-pump pressure detector P1 exceeds a certain range (i.e., is more negative than the certain range), it can damage blood cells. To prevent excessively negative pressure at the pre-pump pressure detector P1, the ECMO system control component can incorporate a P1 pressure stabilization mode. Physicians can set a safety value for the pre-pump pressure detector P1 based on the patient's condition. If the P1 pressure stabilization safety value is outside the pressure alarm range, the ECMO user (e.g., the physician) should set an appropriate P1 pressure alarm range according to the "Extracorporeal Life Support Organization (ELSO) General Guidelines for All ECMO Cases," such as 0 to -400 mmHg. The P1 pressure stabilization safety value can be set to any value between -1 and -400 mmHg, depending on the patient's condition. For example, the physician can set the P1 pressure stabilization safety value using the ECMO system's display panel (e.g., a screen).

Similarly, if a P2 and/or P3 pressure stabilization mode function is added to the ECMO system, and the pressure change trend in the ECMO system is such that increasing the motor speed decreases the P1 pressure value and increases the P2 / P3 pressure values; then decreasing the motor speed increases the P1 pressure value and decreases the P2 / P3 pressure value. When the P1 pressure value is used to determine the condition for entering the pressure mode because the P1 pressure value is less than the first safety value, then the P1 pressure value is used to determine the condition for exiting the pressure mode, i.e., when the P1 pressure value is greater than or equal to the first safety value. When the P2 pressure value is used to determine the condition for entering the pressure mode because the P2 pressure value is greater than the second safety value, then the P2 pressure value is used to determine the condition for exiting the pressure mode, i.e. when the P2 pressure value is less than or equal to the second safety value. When the P3 pressure value is used to determine the condition for entering the pressure mode, the P3 pressure value is greater than the third safety value, and the P3 pressure value is used to determine the condition for exiting the pressor mode, i.e. when the P3 pressure value is less than or equal to the third safety value.

In one possible implementation, in S101, turning on the switch of the pressure stabilization mode may include:
when receiving the command to start the pressure stabilization mode, the pressure stabilization mode is turned on if the following conditions are met at the same time:
the power source of the extracorporeal membrane oxygenation system is in operation;
the pressure detectors of the extracorporeal membrane oxygenation system are all connected correctly;
the flow detector of the extracorporeal membrane oxygenation system is correctly connected.

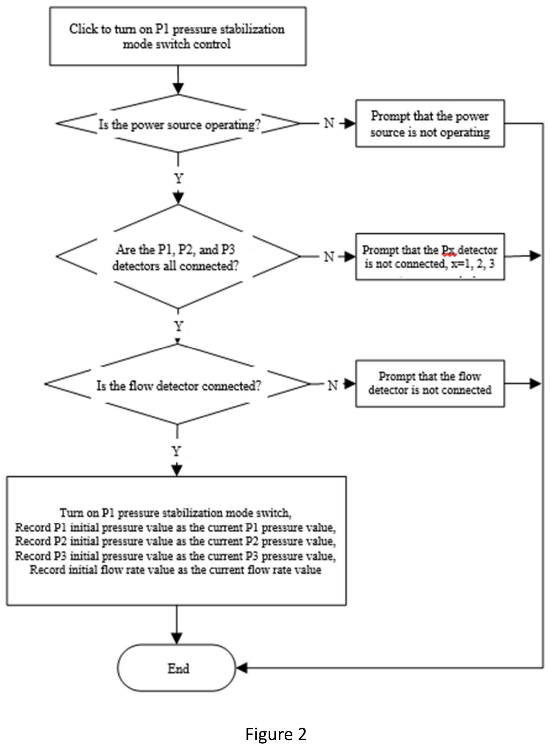

For example, Figure 2 is a flow chart of a switch for turning on the P1, P2, or P3 pressure stabilization mode provided in an embodiment of the present application (with P1 solely depicted in Figure 2, although P2 or P3 could be substituted therefor). As shown in Figure 2, an option for turning on the switch for the P1 pressure stabilization mode can be provided at the advanced settings on the operation interface of the display panel of the ECMO system. The switch for turning on the P1 pressure stabilization mode needs to meet the above-mentioned conditions for turning on the pressure stabilization mode. If the P1 pressure stabilization mode switch is turned on, the display panel will display the switch for the P1 pressure stabilization mode at the P1 parameter display. If the P1 pressure stabilization mode needs to be set, the icon of the switch can be clicked to quickly open and close the setting interface. The purpose of this design is that since turning on the P1 pressure stabilization mode is highly subjective, the switch for turning off the P1 pressure stabilization mode can be quickly found to prevent human errors such as unfamiliar operation and accidental turning on, so as to improve the safety of the ECMO system. Before entering the pressure stabilization mode, the currently measured pressure value is set to the initial pressure value, and the currently measured flow value is set to the initial flow value.

In one possible implementation, the condition for entering the pressure stabilization mode in S102 includes: the switch of the pressure stabilization mode is turned on, and at least one of the following is satisfied: the pressure value of the pre-pump pressure detector of the extracorporeal membrane oxygenation system is less than a first safety value, the pressure value of the pre-membrane pressure detector of the extracorporeal membrane oxygenation system is greater than a second safety value, and the pressure value of the post-membrane pressure detector of the extracorporeal membrane oxygenation system is greater than a third safety value.

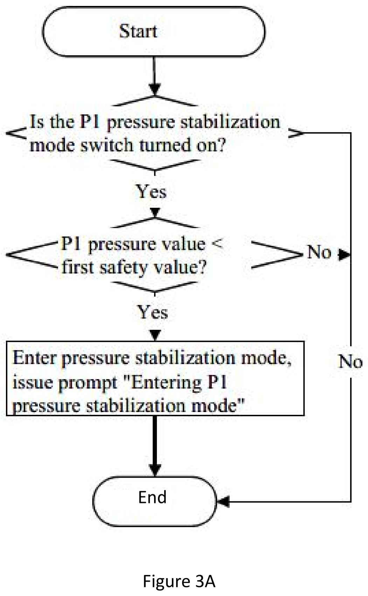

For example, Figure 3A is a flow chart of entering the P1 pressure stabilization mode provided in an embodiment of the present application. As shown in Figure 3A, when the switch of the P1 pressure stabilization mode is turned on, the display panel of the ECMO system will read the status of the pressure detector P1 at a fixed frequency. When it is determined that the above-mentioned conditions for entering the P1 pressure stabilization mode are met, it will enter the P1 pressure stabilization mode and start to automatically adjust the pressure. It should be noted that if an abnormality is found after entering the P1 pressure stabilization mode, the display panel (not shown) will issue an alarm prompt. In addition, in the P1 pressure stabilization mode, the display color, size, etc. of the speed font can be clearly distinguished from the speed font under normal circumstances to prompt medical staff that the ECMO system has entered the P1 pressure stabilization mode.

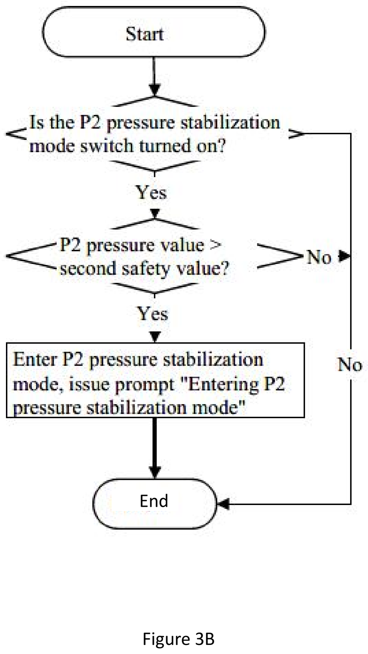

Similarly, Figure 3B is a flow chart of entering the P2 pressure stabilization mode or the P3 pressure stabilization mode provided in an embodiment of the present application. As shown in Figure 3B, taking the P2 pressure stabilization mode as an example, when the switch of the P2 pressure stabilization mode is turned on, the display panel of the ECMO system will read the status of the pressure detector P2 at a fixed frequency. When it is determined that the conditions for entering the P2 pressure stabilization mode are met, the system will enter the pressure stabilization mode and start to automatically adjust the pressure. It should be noted that if an abnormality is found after entering the P2 pressure stabilization mode, the display panel (not shown) will issue an alarm prompt. In addition, in the P2 pressure stabilization mode, the display color and size of the speed font can be clearly distinguished from the speed font under normal circumstances to prompt medical personnel that the ECMO system has entered the P2 pressure stabilization mode. The specific implementation of entering the P3 pressure stabilization mode is the same as that of entering the P2 pressure stabilization mode, and the embodiments of the present application will not be repeated here.

It should be noted that the conditions for entering the P1 pressure stabilization mode, the conditions for entering the P2 pressure stabilization mode, and the conditions for entering the P3 pressure stabilization mode can be implemented independently, as shown in Figures 3A and 3B above, or can be implemented in combination, and the embodiments of the present application are not limited thereto. For example, the pressure stabilization mode may be entered only when at least two of the conditions for entering the P1 pressure stabilization mode, the conditions for entering the P2 pressure stabilization mode, and the conditions for entering the P3 pressure stabilization mode are met.

In one possible implementation, S104, adjusting the speed of the power source of the extracorporeal membrane oxygenation system to a target speed based on the speed change, the flow change, and the pressure change, includes:
determining that the speed change is greater than or equal to a speed threshold, and that the speed change is positively correlated with the flow change and the pressure change, respectively;
determining that the flow rate change is greater than or equal to a flow rate threshold, and the pressure change is greater than or equal to a pressure threshold;
performing a proportional-integral-derivative (PID) adjustment according to a pressure value recorded by at least one of the pre-pump pressure detector, a pre-membrane pressure detector, and a post-membrane pressure detector of the extracorporeal membrane oxygenation system and at least one corresponding safety value to generate a first speed;
if the first speed is greater than or equal to the minimum load speed, setting the first speed as the target speed, the minimum load speed being determined according to the load capacity of the power source in the extracorporeal membrane oxygenation system and the expected load;
the target speed is sent to the control component of the power source.

It should be noted that, when calculating the first rotational speed, only the pressure value recorded by one pressure detector may be used, or the pressure values recorded by multiple pressure detectors may be used.

Using the pressure values recorded by the pre-pump pressure detector P1 and the pre-membrane pressure detector P2 as an example, in one possible implementation method, two PIDs can be set, such as PID1 and PID2. The pressure value P1 recorded by the pre-pump pressure detector P1 and the first safety value can be input into PID1, and the pressure value P2 and the second safety value can be input into PID 2. PID 1 and PID2 respectively output an intermediate value of the first speed, such as the first speed A and the first speed B. The finally generated first speed can be the average value of the first speed A and the first speed B.

It should be noted that when three pressure values or any combination of two pressure values other than P1 and P2 are used to calculate the first speed, the implementation method is the same as the implementation method of generating the first speed using the pressure values P1 and P2.

Optionally, the speed threshold is selected from a hydraulic characteristic curve of the extracorporeal membrane oxygenation system and is a minimum load speed change that causes a recognizable change in pressure under any state.

Optionally, the pressure change is the difference between a first pressure value recorded by at least one of the pre-pump pressure detector, pre-membrane pressure detector, and post-membrane pressure detector of the extracorporeal membrane oxygenation system after the speed of the power source of the extracorporeal membrane oxygenation system is adjusted using the target speed, and a second pressure value recorded by at least one of the pre-pump pressure detector, pre-membrane pressure detector, and post- membrane pressure detector of the extracorporeal membrane oxygenation system before the speed of the power source of the extracorporeal membrane oxygenation system is adjusted using the target speed.

It should be noted that when multiple pressure detectors are used, the pressure changes should be calculated individually because the pre-pump pressure, pre-membrane pressure, and post-membrane pressure can change independently. For example, a change in P1 is calculated using a first pre-pump pressure and a second pre-pump pressure..

Optionally, the flow rate change is the difference between a first flow rate value recorded by a flow detector of the extracorporeal membrane oxygenation system after the speed of the power source of the extracorporeal membrane oxygenation system is adjusted using the target speed, and a second flow rate value recorded by the flow detector of the extracorporeal membrane oxygenation system before the speed of the power source of the extracorporeal membrane oxygenation system is adjusted using the target speed.

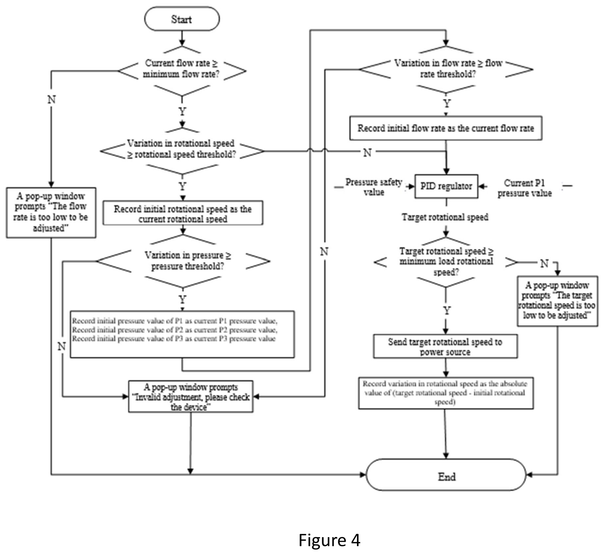

Figure 4 sets forth a process for a pressure stabilization adjustment for an extracorporeal membrane oxygenation (ECMO) system, wherein the flowchart describes the logic for determining whether and how to adjust the rotational speed of the system's power source based on monitored flow rate, rotational speed, and pressure variations.

The process begins by evaluating whether the current flow rate meets or exceeds a minimum flow rate threshold. In the event that the current flow rate falls below the minimum threshold, the system displays a pop-up window prompting "The flow rate is too low to be adjusted," and the process terminates without further action. This safety check ensures the system does not attempt adjustments when flow conditions are inadequate for safe operation. Conversely, if the current flow rate is adequate, the process proceeds to evaluate system variations across multiple parameters.

The system then evaluates three key parameters in sequence to determine whether the system has experienced sufficient changes to warrant recording new baseline values. First, the system evaluates whether the variation in rotational speed is greater than or equal to a rotational speed threshold. Upon satisfaction of this condition, the system records the initial rotational speed as the current rotational speed, updating this baseline value accordingly. Second, the system determines whether the variation in pressure is greater than or equal to a pressure threshold. If so, the system records the initial pressure values for all three pressure monitoring points, specifically updating the initial pressure value of P1 as the current P1 pressure value, the initial pressure value of P2 as the current P2 pressure value, and the initial pressure value of P3 as the current P3 pressure value. Third, the system determines whether the variation in flow rate is greater than or equal to a predefined flow rate threshold. If this condition is satisfied, the system records the initial flow rate as the current flow rate, thereby establishing a new baseline for subsequent comparisons.

If the conditions are not satisfied in the second (pressure) and third (flow rate) operations, then the system displays a pop-up window prompting "Invalid adjustment, please check the device." If the condition for the first operation (speed) is not met, then the system advances to the point right after where the system records the initial flow rate as the current flow rate, as mentioned above.

At this point, the process flows to a PID (Proportional-Integral-Derivative) regulator component. The PID regulator receives two inputs: the current P1 pressure value and a pressure safety value. Using these inputs, the PID regulator calculates a target rotational speed for the power source, which represents the adjusted speed necessary to stabilize pressure within the system.

The system then evaluates whether the target rotational speed calculated by the PID regulator is greater than or equal to a minimum load rotational speed. In the event that the target rotational speed falls below the minimum load rotational speed, the system displays a pop-up window prompting "The target rotational speed is too low to be adjusted," and the process terminates without making any adjustments to the power source. However, if the target rotational speed meets or exceeds the minimum load rotational speed, the process proceeds and the system sends the target rotational speed to the power source. Then, the system records the variation in rotational speed as the absolute value of the difference between the target rotational speed and the initial rotational speed. After that, the process terminates.

Furthermore, the method may further include S105:
S105: If one or more of the following conditions are met, exit the pressure stabilization mode or do not enter the pressure stabilization mode:
the flow detector of the extracorporeal membrane oxygenation system is faulty or not connected;
the pressure detector of the extracorporeal membrane oxygenation system is faulty or not connected;
the flow rate change is less than the flow rate threshold for M consecutive times, where M is a positive integer and M>1;
the pressure change is less than the pressure threshold for N consecutive times, where N is a positive integer and N>1;
the power source of the extracorporeal membrane oxygenation system is not operating;
the pressure value recorded by the pre-pump pressure detector of the extracorporeal membrane oxygenation system is greater than or equal to the first safety value;
the pressure value recorded by the pre-membrane pressure detector of the extracorporeal membrane oxygenation system is less than or equal to the second safety value;
the pressure value recorded by the post-membrane pressure detector of the extracorporeal membrane oxygenation system is less than or equal to the third safety value;
an instruction to intervene in the rotation speed of the power source of the extracorporeal membrane oxygenation system is received from the outside.

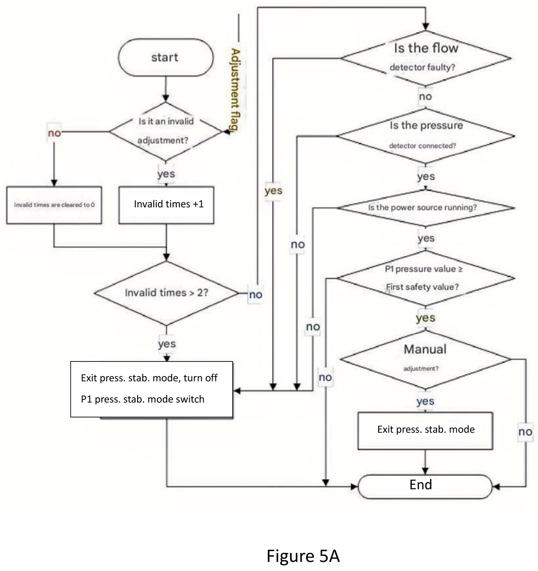

For example, Figure 5A is a schematic diagram of a process for exiting P1 pressure stabilization mode, provided in an embodiment of the present application. In general, as shown in Figure 5A, after turning on the P1 pressure stabilization mode switch, the ECMO system status can be monitored in real time. If any abnormality is detected, the system automatically exits or does not enter P1 pressure stabilization mode, thereby further improving safety and efficiency.

More specifically, in the illustrated embodiment, Figure 5A illustrates the process for monitoring and exiting the P1 pressure stabilization mode in an ECMO system. The process begins with an adjustment flag that initiates a continuous monitoring sequence to evaluate whether conditions warrant exiting the pressure stabilization mode.

The system then evaluates whether the current adjustment being made is valid or invalid. If the adjustment is determined to be valid, the system clears the invalid times counter back to zero and continues on in the process. However, if the adjustment is invalid, the system increments the invalid times counter by one and then checks if the invalid times counter is greater than two. If so, the system proceeds to exit the pressure stabilization mode and turns off the P1 pressure stabilization mode switch. If not, the system continues with further evaluations.

During the further evaluations, the system first checks whether the flow detector is functioning properly. If a fault is detected in the flow detector, the system proceeds to exit the pressure stabilization mode and turn off the P1 pressure stabilization mode switch. If the flow detector is operating normally, the next checkpoint verifies whether the pressure detector is properly connected to the system. If the pressure detector is disconnected or malfunctioning, the system exits the pressure stabilization mode and turns off the P1 pressure stabilization mode switch. If the pressure detector is functioning correctly, the process continues by checking whether the power source or motor is currently running.

If the power source is not operational, the system proceeds to exit the pressure stabilization mode and turn off the P1 pressure stabilization mode switch. If the power source is confirmed to be running, the system then checks whether the P1 pressure value has reached or exceeded the first safety value. If the P1 pressure value is still below the first safety value, the system returns to finish without exiting the stabilization mode, allowing continued pressure regulation. However, if the P1 pressure value has reached or exceeded the first safety value, the system performs one final check to determine whether manual adjustment of the rotational speed is occurring. If manual intervention is detected, the system exits the pressure stabilization mode. If no manual adjustment is detected, the system simply exits the pressure stabilization mode, concluding the monitoring cycle.

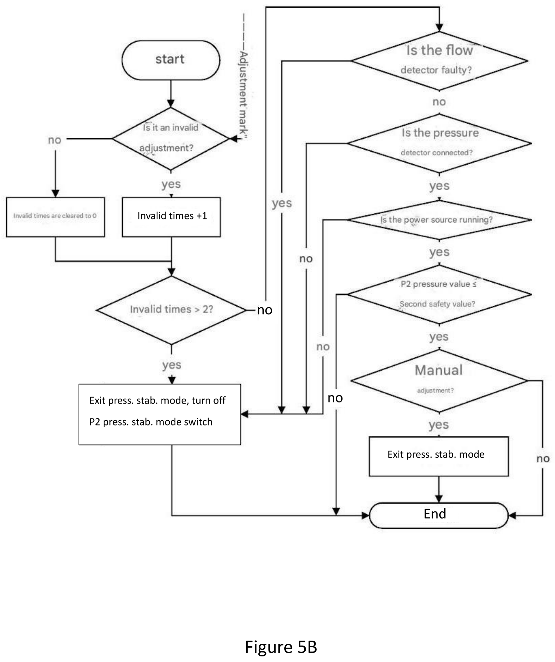

For example, Figure 5B is a schematic diagram of a process for exiting P2 pressure stabilization mode, provided in an embodiment of the present application. In general, as shown in Figure 5B, after turning on the P2 pressure stabilization mode switch, the ECMO system status can be monitored in real time. If any abnormality is detected, the system automatically exits or does not enter P2 pressure stabilization mode, thereby further improving safety and efficiency.

More specifically, in the illustrated embodiment, Figure 5B illustrates the process for monitoring and exiting the P2 pressure stabilization mode in an ECMO system. The process begins with an adjustment flag that initiates a continuous monitoring sequence to evaluate whether conditions warrant exiting the pressure stabilization mode.

The system then evaluates whether the current adjustment being made is valid or invalid. If the adjustment is determined to be valid, the system clears the invalid times counter back to zero and continues on in the process. However, if the adjustment is invalid, the system increments the invalid times counter by one and then checks if the invalid times counter is greater than two. If so, the system proceeds to exit the pressure stabilization mode and turns off the P2 pressure stabilization mode switch. If not, the system continues with further evaluations.

During the further evaluations, the system first checks whether the flow detector is functioning properly. If a fault is detected in the flow detector, the system proceeds to exit the pressure stabilization mode and turn off the P2 pressure stabilization mode switch. If the flow detector is operating normally, the next checkpoint verifies whether the pressure detector is properly connected to the system. If the pressure detector is disconnected or malfunctioning, the system exits the pressure stabilization mode and turns off the P2 pressure stabilization mode switch. If the pressure detector is functioning correctly, the process continues by checking whether the power source or motor is currently running.

If the power source is not operational, the system proceeds to exit the pressure stabilization mode and turn off the P2 pressure stabilization mode switch. If the power source is confirmed to be running, the system then checks whether the P2 pressure value has reached or subceeded the second safety value. If the P2 pressure value is still above the second safety value, the system returns to finish without exiting the stabilization mode, allowing continued pressure regulation. However, if the P2 pressure value has reached or subceeded the second safety value, the system performs one final check to determine whether manual adjustment of the rotational speed is occurring. If manual intervention is detected, the system exits the pressure stabilization mode. If no manual adjustment is detected, the system simply exits the pressure stabilization mode, concluding the monitoring cycle.

It should be noted that the three pressure value judgment conditions for exiting the pressure stabilization mode can be implemented independently, as shown in Figures 5A and 5B, or can be implemented in combination, such as judging whether the conditions for exiting the pressure stabilization mode are met based on two or more pressure values at the same time.

In addition, the above-mentioned P1 pressure stabilization mode, P2 pressure stabilization mode, and P3 pressure stabilization mode can also be collectively referred to as pressure stabilization mode. In this case, the conditions for entering the pressure stabilization mode and the conditions for exiting the pressure stabilization mode can be implemented in combination. For example, the pre-pump pressure value P1 can be used to determine whether to enter the pressure stabilization mode, as shown in Figure 3A, and the pre-membrane pressure value P2 can be used to determine whether to exit the pressure stabilization mode, as shown in Figure 5B. For another example, the pre-membrane pressure value P2 can be used to determine whether to enter the pressure stabilization mode, as shown in Figure 3B, and the pre-pump pressure value P1 can be used to determine whether to exit the pressure stabilization mode, as shown in Figure 5A. The embodiments of the present application do not limit the combination of the judgment conditions for entering and exiting the pressure stabilization mode.

It should be noted that in actual applications, various detectors such as speed, pressure, and flow can include multiple sensors. For example, three different pressure detectors P1, P2, and P3 can be set before the pump, before the membrane, and after the membrane of the ECMO system respectively (for a total of nine sensors). In addition, multiple types of detectors can be set to provide more accurate and comprehensive judgment conditions to further improve the accuracy and reliability of the judgment conditions.

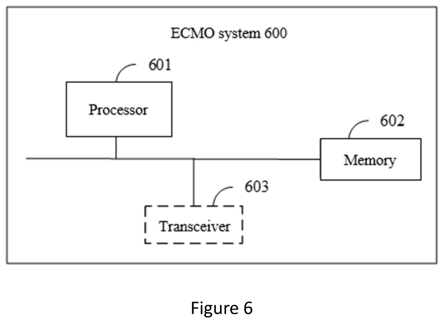

For example, Figure 6 is a schematic diagram of the structure of an extracorporeal membrane oxygenation system provided in an embodiment of the present application. As shown in Figure 6, the system 600 includes a processor 601 and a memory 602. The memory 602 stores a program or instruction. When the program or instruction is executed by the processor 601, the extracorporeal membrane oxygenation system 600 executes the method for managing the pressure stabilization mode of the extracorporeal membrane oxygenation system as described in any of the implementations of the above method embodiments.

In addition, the ECMO system 600 can also be called an ECMO device, an ECMO apparatus, etc. In addition to the processor 601 and the memory 602, it can also include various parts or components such as pipelines, power sources, input and output devices, and a transceiver 603, which are not limited in the embodiments of the present application.

The present application provides a computer-readable storage medium storing a program or instruction. When a computer reads and executes the program or instruction, the computer executes the method for managing the pressure stabilization mode of an extracorporeal membrane oxygenation system as described in any of the above method embodiments.

Based on the management method, system and computer-readable storage medium of the pressure stabilization mode of the extracorporeal membrane oxygenation system provided in the embodiments of the present application, the target speed can be generated by comprehensively considering the speed change of the power source of the ECMO system and the flow change and pressure change recorded by various detectors. The target speed is used to adjust the motor speed of the ECMO system so as to keep the flow and pressure of the ECMO system within a safe range. This can solve the problem of adjusting the motor speed of the ECMO system only according to the pressure value recorded by the pressure detector, resulting in excessively high or low pressure values and thus posing a safety risk, thereby improving the safety of the ECMO system.

Finally, it should be noted that the above embodiments are only used to illustrate examples of the technical solutions provided by the embodiments of the present application, and are not intended to limit them. Although the present application has been described in detail with reference to the aforementioned embodiments, those skilled in the art should understand that they can still modify the technical solutions described in the aforementioned embodiments, or make equivalent replacements for some or all of the technical features therein. These modifications or replacements do not deviate the essence of the corresponding technical solutions from the scope of the technical solutions of the embodiments of the present application.

## Claims

1. A method for managing a pressure stabilization mode of an extracorporeal membrane oxygenation system that comprises a pump, a membrane oxygenator, a flow detector, and at least one pressure detector fluidly connected together with tubing, the method comprising:
turning on a switch for the pressure stabilization mode of the extracorporeal membrane oxygenation system;
determining whether a condition for entering the pressure stabilization mode is met;
entering the pressure stabilization mode in response to determining that the condition is met;
acquiring a rotation speed change of a power source of the pump of the extracorporeal membrane oxygenation system, a flow rate change of the flow detector of the extracorporeal membrane oxygenation system, and a pressure change of the at least one pressure detector of the extracorporeal membrane oxygenation system; and
adjusting the rotation speed of the power source of the extracorporeal membrane oxygenation system to a target speed according to the rotation speed change, the flow rate change, and the pressure change to keep the flow rate and pressure within a safe range.

2. The method according to claim 1, wherein the at least one pressure detector includes a pre-pump pressure detector upstream of the pump, a pre-membrane pressure detector between the pump and the membrane oxygenator, and a post-membrane pressure detector downstream of the membrane oxygenator, and the method is **characterized in that** the conditions for entering the pressure stabilization mode include:
the switch of the pressure stabilization mode is turned on, and
at least one of the following is satisfied:
the pressure value of the pre-pump pressure detector of the extracorporeal membrane oxygenation system is less than a first safety value;
the pressure value of the pre-membrane pressure detector of the extracorporeal membrane oxygenation system is greater than a second safety value; and
the pressure value of the post-membrane pressure detector of the extracorporeal membrane oxygenation system is greater than a third safety value.

3. The method according to claim 1, wherein adjusting the speed of the power source of the extracorporeal membrane oxygenation system to the target speed according to the rotation speed change, the flow rate change, and the pressure change comprises:
determining that the rotation speed change is greater than or equal to a speed threshold, and that the rotation speed change is positively correlated with the flow change and the pressure change, respectively;
determining that the flow rate change is greater than or equal to a flow rate threshold, and the pressure change is greater than or equal to a pressure threshold;
performing proportional-integral-differential adjustment according to a pressure value and a safety value recorded by at least one of the at least one pressure detector to generate a first rotational speed, wherein the at least one pressure detector includes a pre-pump pressure detector, a pre-membrane pressure detector, and a post-membrane pressure detector of the extracorporeal membrane oxygenation system;
setting the first speed as the target speed in response to the first speed being greater than or equal to a minimum load speed, wherein the minimum load speed is determined according to the load capacity and expected load of the power source in the extracorporeal membrane oxygenation system; and
sending the target speed to a control component of the power source.

4. The method according to claim 3, wherein the speed threshold is selected from the hydraulic characteristic curve of the extracorporeal membrane oxygenation system and is the minimum load speed change that will cause a recognizable pressure change in any state.

5. The method according to claim 3, wherein the at least one pressure detector includes a pre-pump pressure detector upstream of the pump, a pre-membrane pressure detector between the pump and the membrane oxygenator, and a post-membrane pressure detector downstream of the membrane oxygenator, and the method is **characterized in that** the pressure change is a difference between a first pressure value recorded by at least one of the pre-pump pressure detector, the pre-membrane pressure detector, and the post-membrane pressure detector of the extracorporeal membrane oxygenation system after the speed of the power source of the extracorporeal membrane oxygenation system is adjusted using the target speed, and a second pressure value recorded by at least one of the pre-pump pressure detector, the pre-membrane pressure detector, and the post-membrane pressure detector of the extracorporeal membrane oxygenation system before the speed of the power source of the extracorporeal membrane oxygenation system is adjusted using the target speed.

6. The method according to claim 3, wherein the flow rate change is a difference between a first flow rate value recorded by the flow detector of the extracorporeal membrane oxygenation system after the speed of the power source of the extracorporeal membrane oxygenation system is adjusted using the target speed and a second flow rate value recorded by the flow detector of the extracorporeal membrane oxygenation system before the speed of the power source of the extracorporeal membrane oxygenation system is adjusted using the target speed.

7. The method according to claim 1, wherein adjusting the speed of the power source of the extracorporeal membrane oxygenation system to a target speed based on the speed change, flow change, and pressure change comprises:
determining that the speed change is less than a speed threshold;
generating a second rotational speed;
setting the second speed as the target speed in response to the second speed being greater than or equal to the minimum load speed, wherein the minimum load speed is determined according to the load capacity and expected load of the power source of the extracorporeal membrane oxygenation system; and
sending the target speed to a control component of a power source of the extracorporeal membrane oxygenation system.

8. The method according to any one of claims 1 to 7, wherein the pressure stabilization mode is exited or not entered in response to one or more of the following conditions being met:
the flow detector of the extracorporeal membrane oxygenation system is faulty or not connected;
the at least one pressure detector of the extracorporeal membrane oxygenation system is faulty or not connected;
the flow rate change is less than the flow rate threshold for M consecutive times, where M is a positive integer and M>1;
the pressure change is less than the pressure threshold value N times in a row, where N is a positive integer and N>1;
the power source of the extracorporeal membrane oxygenation system is not operating;
the pressure value recorded by the pre-pump pressure detector of the extracorporeal membrane oxygenation system is greater than or equal to the first safety value;
the pressure value recorded by the pre-membrane pressure detector of the extracorporeal membrane oxygenation system is less than or equal to the second safety value;
the pressure value recorded by the post-membrane pressure detector of the extracorporeal membrane oxygenation system is less than or equal to the third safety value; and
an instruction to intervene in the rotation speed of the power source of the extracorporeal membrane oxygenation system is received from the outside.

9. An extracorporeal membrane oxygenation system further comprising a processor and a memory, wherein the memory stores a program or instruction, and when the program or instruction is executed by the processor, the extracorporeal membrane oxygenation system executes the method for managing a pressure stabilization mode of the extracorporeal membrane oxygenation system according to any one of claims 1 to 8.

10. A computer-readable storage medium, **characterized in that** it stores a program or instruction, which, when a computer reads and executes the program or instruction, enables the computer to execute the method for managing the pressure stabilization mode of the extracorporeal membrane oxygenation system according to any one of claims 1 to 8.
